# EUROPEAN PATENT SPECIFICATION

(11) **EP 1 968 482 B1**
(45) Date of publication and mention of the grant of the patent: **17.06.2009**
(21) Application number: 06842690.7
(22) Date of filing: 28.12.2006
(51) Int. Cl.: A61F 2/00

(54) **Artificial contractile tissue**
Künstliches kontraktiles Gewebe
Tissu contractile artificiel

(30) Priority: 04.01.2006 WO PCT/IB2006/050033
(43) Date of publication of application: 17.09.2008
(73) Proprietor: Nanopowers S.A., 1012 Lausanne (CH)
(72) Inventor: TOZZI, Piergiorgio, CH-1012 Lausanne (CH); HAYOZ, Daniel, CH-1752 Villars-sur-Glâne (CH); VON SEGESSER, Ludwig, CH-1005 Lausanne (CH)
(74) Representative: GLN
(86) International application number: PCT/IB2006/055044
(87) International publication number: WO 2007/077513

(56) References cited:
- WO-A-02/19917
- US-A- 6 076 013
- US-A1- 2003 153 949
- US-A1- 2004 034 271
- US-A1- 2004 064 014
- US-A1- 2005 020 871
- US-A1- 2005 148 814

## Description

### Technical Field

The present invention relates to an artificial contractile tissue generally devised to be used in the medical field. Such a tissue may be advantageously used to assist muscular contraction, in particular atrial contraction of patients with atrial fibrillation.

### Background of the invention

Artificial supports to assist muscular contraction are disclosed in Japanese patent applications JP 2001112796 and JP 7008515.

The devices described in this prior art act as muscle fibers and are therefore not adapted to completely replace a muscle tissue.

US patent application US 2005/0020871 discloses an artificial beating tissue based on nanotechnology actuators as source of one or more spatially oriented forces which are used to exert an extra pressure on the cardiac region to be assisted. To this effect, a network of contractile elements connected with longitudinal elements is provided. The network is embedded in an elastomeric material. Activation of the contractile elements causes a reduction in their length that is associated to the contraction of the web.

US 2003/0153949 discloses an artificial contractile tissue in a form of a cardiac harness, to apply a compressive force over a range of elastic deformation of the harness. The harness can be shifted to a second range of deformation by application of a stimulus.

### Summary of the invention

The objective of the present invention is to provide an improved artificial contractile tissue.

This objective has been reached according to the present invention by an artificial contractile tissue comprising a rigid structure forming a closed line and several fibers of variable length which are fixed at their ends to two separate points of the structure and which are distributed across said structure in such a way to create a dome. The fibers are made of a contractile material which can be activated by an activator, e.g. an electric current/voltage, in such a way as to provide a tissue in a rest or in an activated position, the rest position being defined with non-rectilinear fibers and the activated position being defined with fibers of reduced length. The transition from the rest towards the activated position or vice-versa is defined by a fiber movement along a lateral direction which is perpendicular with respect to the fiber length, so that the dome gets closer to the rigid structure.

The closed line may be comprised in a plane and may form any shape, regular or not, for instance a circle, an ellipse, a square or a triangle.

In a preferred embodiment, the structure has an annular shape and each fiber forms a diameter of the annular structure. This means that all fibers are crossing each other at the center of the annular structure. At this point, the fibers are advantageously glued to each other.

In another embodiment, the structure has an annular shape and each fiber forms a loop around a central piece, called pivot hereafter, which is located at the center of the annular structure.

On one or both sides of the tissue, a membrane, e.g. made of silicone, may cover the fibers.

When using a planar structure, at rest position, the plane preferably forms an angle of 20 to 35° with the fiber ends.

Advantageously the external surface of the structure comprises a sewing surface, for instance a Dacron™ coating.

If the activator is an electric current an isolating substance preferably covers the fibers. For instance, fibers may be inserted in ePTFE tubes.

Any suitable material can be used for the fibers, in particular Electro Active Polymers (EAP), Electro Active Ceramics (EAC), Shape Memory Alloys (SMA).

SMA undergo changes in shape and hardness when heated or cooled, and do so with great force. The mechanism of the shape memory effect is a diffusionless phase transformation as a solid, in which atoms move cooperatively, often by shear like mechanisms. SMA have a uniform crystal structure that radically changes to a different structure at a specific temperature. When the SMA is below this transition temperature (martensitic state) it can be stretched and deformed without permanent damages. After the SMA has been stretched, if it is heated (i.e. electrically) above its transition temperature (austenite state), the alloy recovers to the un-stretched shape and completely reverses the previous deformation.

Moreover, SMA are capable to lift thousand times their own weight. SMA have the ability to recover from plastic deformation, which is sustained below critical temperature, by heating, and they can work under tension, compression, bending or torsion.

Table 1 below shows a comparison of the properties of materials which may be used for artificial muscles: Electro Active Polymers, Shape Memory Alloys and Electro Active Ceramics.

**Table 1**

| Property | ElectroActive Polymers (EAP) | Shape Memory Alloys (SMA) | Electroactive Ceramics (EAC) |
|---|---|---|---|
| Actuation Displacement | > 10% | < 8% | 0.1-0.3% |
| Force (Mpa) | 10-30 | 700 | 30-40 |
| Reaction speed | µsec | sec | µsec |
| Density | 1-2.5 g/cc | 5 g/cc | 6-8 g/cc |
| Drive voltage | 4-7 V | 4-50 V | 50-800 V |
| Fracture toughness | resilient, elastic | elastic | fragile |

Even if the energetic efficiency of these materials is lower than conventional electric and magnetic pumps (only 5% of the electricity potential for work becomes a usable physical force with 95% lost as heat), their high strength-to-weight ratio, small size and low operating voltages, allow the development of devices that would be difficult or impossible to make using conventional motors with overall better performance than other systems.

A suitable SMA material for the contractible fibers is Nitinol™. In this case the fibers can be stretched by as much as 4% when below the transition temperature, and when heated, they contract, recovering thereby to their original, shorter length with a usable amount of force in the process. Temperature range is 37-50°C.

Other particularly interesting materials are Biometal fibers^{®} (BMF) and Biometal helix^{®} (BMX) commercialized by Toki Corporation Inc., Japan. Those materials are able to reversibly contract upon a controlled heating caused by the supply of an electric current/voltage.

### Brief description of the drawings

The invention is discussed below in a more detailed way with examples illustrated by the following figures :

Figure 1 shows a front view of a first embodiment of the invention.

Figure 2 shows a side view of the embodiment of figure 1.

Figure 3 shows the embodiment of figure 1 in a rest position.

Figure 4 shows the embodiment of figure 1 in an activated position.

Figures 5A and 5B show a second embodiment of the invention in which a central pivot avoids contractile fibers crossing each others.

Figures 6A and 6B show an enlargement of the pivot of figure 5.

Figure 7 shows a first example of a contractile tissue.

Figure 8 shows a second example where the contractile fibers are knitted in a flexible sheet, not part of the invention.

Figures 9A and 9B show the working principle of another examples.

Figure 10 shows the tissue of the first and second examples in a rest and in an activated position.

List of references used in the figures :
a) fiber
b) annular structure
c) apex
d) membrane
e) sewing surface
f) flexible sheet
g) fiber
h) protrusion
i) groove
j) pivot
k) cap

### Detailed description

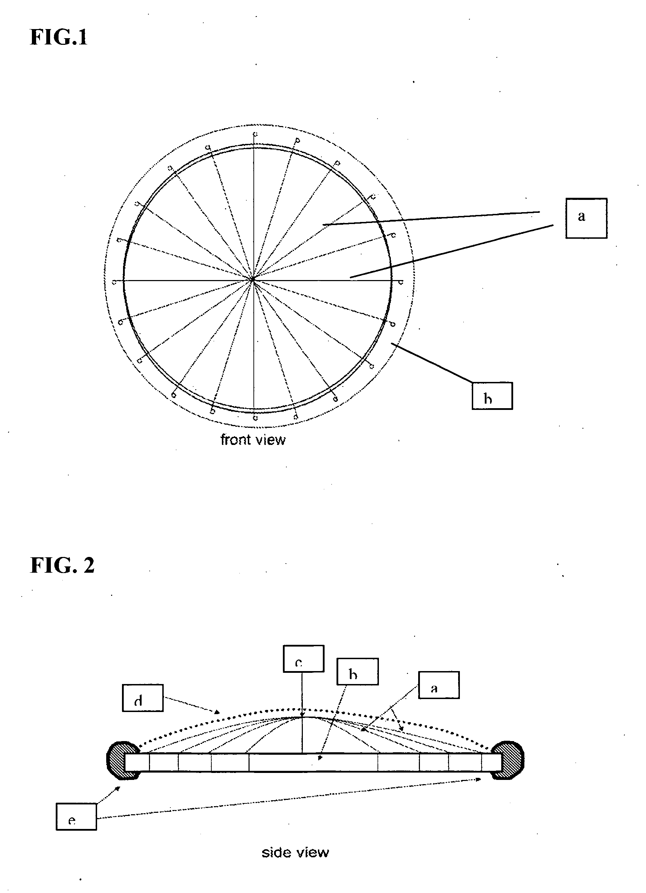

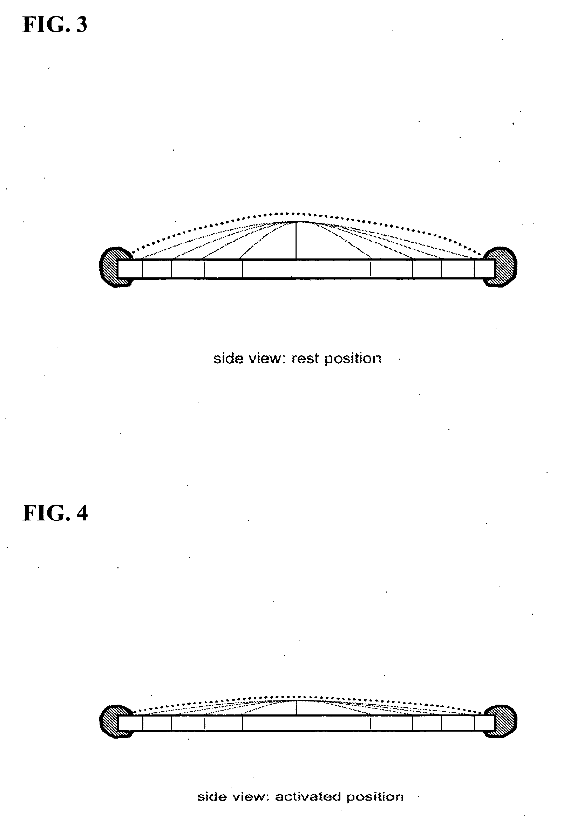

The embodiment illustrated on Figures 1 to 4 is defined by a rigid annular structure b. The fibers a are distributed across the ring and pass through the middle point of the structure b in such a way as to create a dome forming an angle of preferably 20 to 35 ° with respect to the ring plane. The point c where fibers cross each others in the middle point of the ring is the apex of the dome. When an electric current/voltage is applied to the fibers a, their length is reduced and the apex c gets closer to the ring plane of the ring as represented in figure 2. When the dome is applied on the surface of the upper chamber of the heart (atrium), its electrically activated movement pushes the wall of the atrium and its content (the blood). The blood is therefore forced to move into the ventricle. This is the mechanical support to the blood circulation.

The ring may be made of plastic, e.g. Delrin™ and may have other shapes than a circular (ellipse, eight shape, etc...).

Bench tests have demonstrated that a 55 mm dome made of BMX200^{®} can pump 80 ml of water against a pressure of 15 mmHg each time it is activated (contraction). With a rate of contractions of 60 times per minute, a total volume of 480 ml per minute of water may be pumped.

In order to avoid shortcuts, fibers a are isolated, e.g. inserted in ePTFE tubes having an inner diameter which may be of 400µm. The ePTFE tubes are preferably glued together at the apex c.

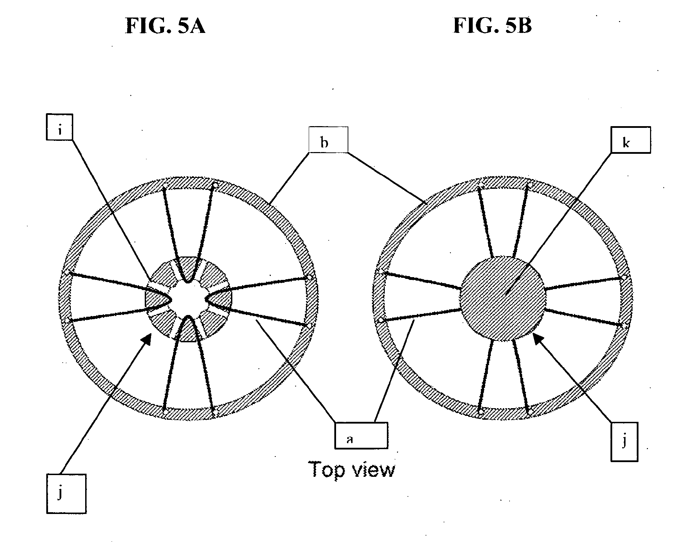

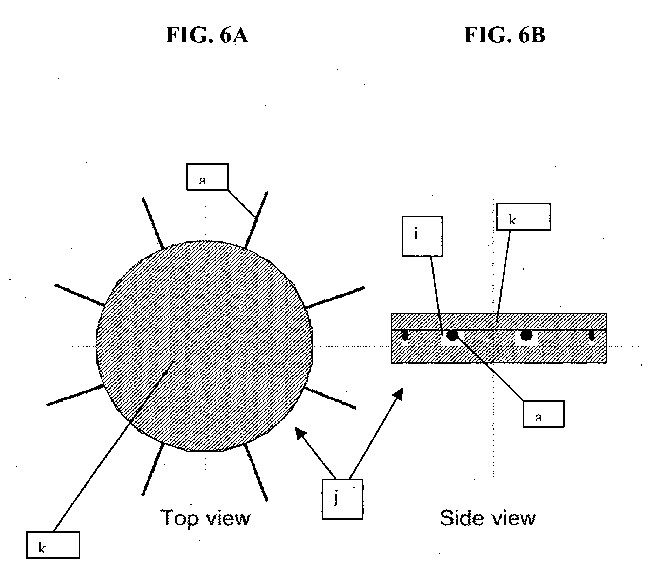

Another mean to avoid shortcuts is to insert a pivot j at the apex c as illustrated in figures 5A to 6B. The pivot j is made of plastic, has a round shape with grooves i on its surface. The fibers a pass into the grooves i forming a loop through the pivot j. The pivot j is furthermore covered by a cap k to ensure proper maintenance of the fibers a in the grooves i.

A thin silicone membrane d, e.g. 100 µm thick, covers the inner and outer part of the dome to provide thermo isolation of the dome thereby reducing the risk of burn lesions on the heart surface.

On the external surface of the ring b, a coating, e.g. made of Dacron™, is fixed to provide a sewing surface e for the connection to the heart.

Advantageously the dome is sutured on the external surface of the upper chamber of the heart (atrium) in the rest position in such a way the atrium completely fills the inner part of the dome.

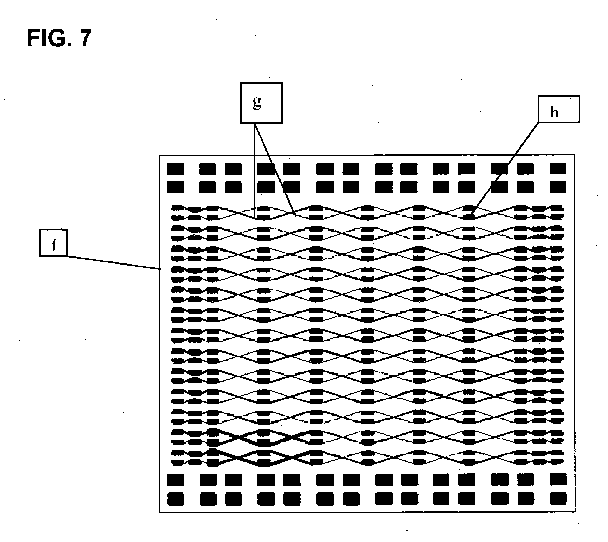

Figures 7 to 10 show an example of an artificial contractile tissue which comprises a flexible sheet.

It should be pointed out at this stage that, "flexible sheet" does not mean "elastomeric material" as disclosed in prior art application US 2005/002071. A flexible sheet as presently defined can be folded but not extended or contracted.

In this example (see figure 7), the artificial muscle essentially consists of a matrix comprising contractible fibers g, e.g. Nitinol fibers, and a flexible sheet f made of polyimide. The matrix includes several protrusions h which may be made of copper and which act as pivots. The fibers g pass around the protrusions h in such a way to create a series of waives. At the matrix edges the fiber ends are fixed, e.g. glued, to the protrusions. Fibers cross each other with an angle of about 40°. In the illustrated embodiment, there are 26 lines of fibers having each 7 waives. Protrusions close to matrix' s edges are used as electric contacts (positive and negative electrodes).

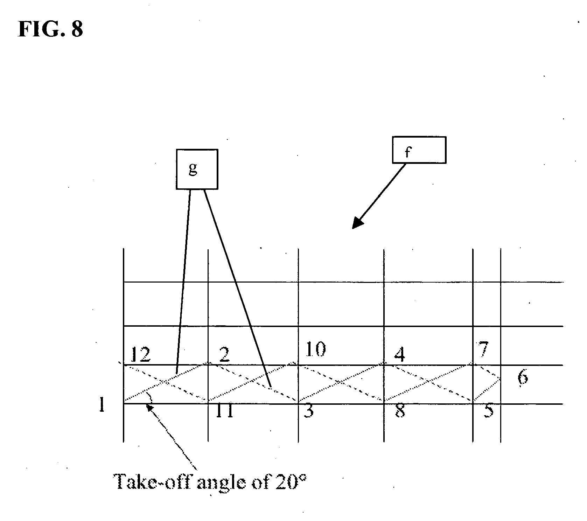

In another example a flexible sheet f is partially and schematically illustrated on figure 8. The sheet f is made of polyester tissue which may be reinforced with Kevlar™ or carbon fibers. Preferably Nitinol fibers (BMF)™ g are knitted in the flexible sheet f, on both sides, in such a way that the sheet f itself avoids shortcuts when an electric current is used to activate the contractile fibers. On figure 8, only one fiber g is illustrated. The numbering shows the successive locations where the fiber g is crossing the sheet f. A full line represents a fiber portion which is above the sheet f while a dashed line represents a fiber portion which is below the sheet f. The contractile fibers g are knitted in the tissue in such a way to create a series of waves as described in the previous embodiment and following the working principle discussed below. The difference is that in the present fibers g are on both sides of the flexible sheet f. Waves are therefore present on both sides of the sheet f and the activation of the fibers g results in a movement of the sheet ends in any desired direction.

Several matrix can be joined together in parallel (to increase the pulling force) and/or serial (to increase the length of the displacement) configuration for different clinical applications.

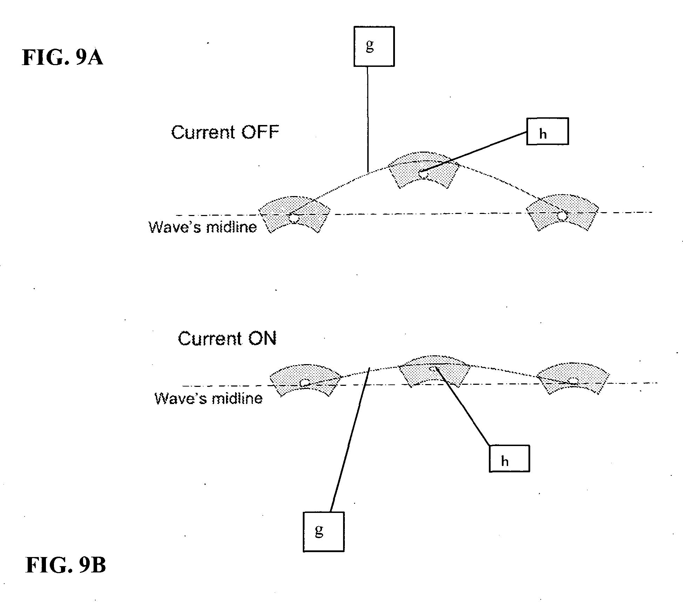

The working principle of the previous cited example will be discussed below and illustrated on figures 9A and 9B.

When electrically activated, the fibers g reach their transitional temperature and may shrink 4% of their length, pulling consequently protrusions h down to the wave' s midline. Because protrusions h are fixed to the matrix, fiber' s activation results in matrix movement.

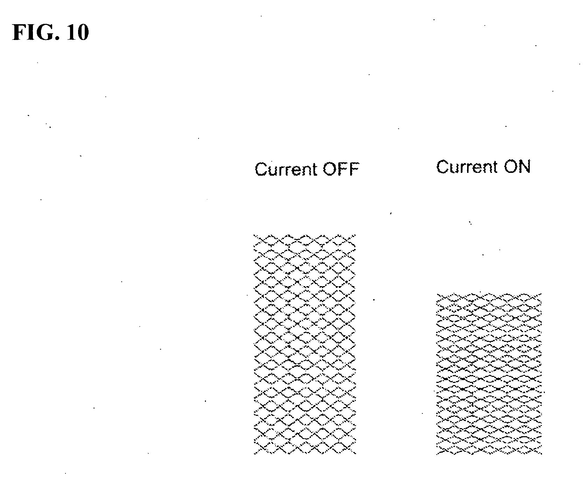

The axe of the movement of the matrix is orthogonal with respect to the fiber movement. Synchronous activation of the 26 fibers causes the matrix shrinking of about 25% as illustrate in figure 10.

The matrix discussed here is able to develop about 240 gf over 6 mm displacement which corresponds to 0.1 W .

A Drive Unit (DU) and a Power Source (PS) are necessary to control and power matrix movement.

The DU is basically a microprocessor that distributes current to fibers. Intensity, width and rate of the electrical stimuli are determined according to the application of the matrix.

The PS may be a rechargeable battery.

The present invention has several applications in the medical field, in particular :

- Artificial Muscle for cardiac assist. In patients suffering from Chronic Atrial Fibrillation, the contractile function of the upper chambers of the heart (called atria) is lost and cannot be restored by any means. The heart is therefore weaker than normal. For instance two domes can be placed around the upper chambers of the heart (atria) and sutured to the external surface of the heart (epicardium). When simultaneously activated (e.g. 1Hz frequency) they squeeze the atrium from outside and replace the natural function of this part of the heart. Such a configuration may offer a force of about 500 g and a displacement of about 25 mm, which corresponds to a power of about 1 W.

The drive unit is similar to that currently used for single chamber cardiac pacemakers: it detects ventricular electrical activity thanks to an epicardial electrode and provides control of current direction, intensity and frequency of activation of contractile elements: the contraction can be synchronous, asynchronous, sequential or others in order to have the most appropriate three dimensional deformations to compress atria and achieve the optimal ventricular filling. Lithium-manganese dioxide batteries (500 mA for 3.2V) provide the power supply and can last for 6h. A percutaneous energy transfer supply can be developed for battery recharge during the night, as routinely done with other ventricular assist devices like LionHeart.

- Treatment of congestive heart failure.

- Treatment of neuromuscular diseases causing paralysis and post traumatic paralysis of lower and/or upper extremities, to increase muscular strength.

- More generally, assisting contraction of an organ (stomach, bladder, urethra, etc.).

## Claims

1. Artificial contractile tissue comprising a rigid structure (b) forming a closed line and several fibers (a) of variable length which are fixed at their ends to two separate points of said structure (b) and which are distributed across said structure (b) in such a way to create a dome; said fibers (a) being made of a contractile material which can be activated by an activator in such a way as to provide a tissue in a rest or in an activated position, the rest position being defined with non-rectilinear fibers (a) and the activated position being defined with fibers (a) of reduced length; the transition from the rest towards the activated position or vice-versa being defined by a fiber movement along a lateral direction which is perpendicular with respect to the fiber length, so that the dome gets closer to said structure (b).

2. Artificial contractile tissue according to claim 1, wherein said closed line is comprised in a plane.

3. Artificial contractile tissue according to any one of the previous claims, wherein said structure (b) has an annular shape, each fiber (a) forming a diameter of said annular structure (b).

4. Artificial contractile tissue according to any one of the previous claims 1 to 2, wherein said structure (b) has an annular shape, each fiber (a) passing into a groove (i) of a central pivot (j) and forming a loop across said pivot (j).

5. Artificial contractile tissue according to any one of the previous claims, furthermore comprising a membrane (d) which covers the fibers (a) on one side of the tissue.

6. Artificial contractile tissue according to claim 5, comprising another membrane which covers the other side of the tissue.

7. Artificial contractile tissue according to any one of the previous claims 2 to 6, wherein at rest position, said plane forms an angle of 20 to 35° with the fiber ends.

8. Artificial contractile tissue according to any one of the previous claims, wherein the external surface of the structure (b) comprises a sewing surface (e).

9. Artificial contractile tissue according to any one of the previous claims, wherein said activator is an electric current/voltage.

## Patentansprüche

1. Künstliches kontraktiles Gewebe, umfassend eine starre Struktur (b), die eine geschlossene Linie bildet, und mehrere unterschiedlich lange Fasern (a), die an ihren Enden an zwei getrennten Stellen der Struktur (b) befestigt sind, und die derart quer über die Struktur (b) verteilt sind, dass sie eine Kuppel bilden; wobei die Fasern (a) aus einem kontraktilen Material ausgebildet sind, das durch einen Aktivator derart aktiviert werden kann, dass ein Gewebe in einer Ruheposition oder in einer aktivierten Position bereitgestellt wird, wobei die Ruheposition durch nicht geradlinige Fasern (a) definiert ist, und die aktivierte Position durch kürzere Fasern (a) definiert ist; wobei der Übergang von der Ruheposition auf die aktivierte Position oder umgekehrt durch eine Faserbewegung in einer seitlichen Richtung definiert wird, die im Verhältnis zur Faserlänge rechtwinklig ist, so dass die Kuppel sich der Struktur (b) nähert.

2. Künstliches kontraktiles Gewebe nach Anspruch 1, wobei die geschlossene Linie in einer Ebene enthalten ist.

3. Künstliches kontraktiles Gewebe nach einem der vorhergehenden Ansprüche, wobei die Struktur (b) eine Ringform aufweist, wobei jede Faser (a) einen Durchmesser der Ringform (b) bildet.

4. Künstliches kontraktiles Gewebe nach einem der vorhergehenden Ansprüche 1 bis 2, wobei die Struktur (b) eine Ringform aufweist, wobei jede Faser (a) in einer Rille (i) eines zentralen Drehzapfens (j) verläuft und eine Schleife über diesen Drehzapfen (j) bildet.

5. Künstliches kontraktiles Gewebe nach einem der vorhergehenden Ansprüche, ferner umfassend eine Membran (d), welche die Fasern (a) auf einer Seite des Gewebes bedeckt.

6. Künstliches kontraktiles Gewebe nach Anspruch 5, umfassend eine andere Membran, welche die andere Seite des Gewebes bedeckt.

7. Künstliches kontraktiles Gewebe nach einem der vorhergehenden Ansprüche 2 bis 6, wobei in einer Ruheposition die Ebene einen Winkel von 20 bis 35° mit den Faserenden bildet.

8. Künstliches kontraktiles Gewebe nach einem der vorhergehenden Ansprüche, wobei die äußere Oberfläche der Struktur (b) eine Nähfläche (e) umfasst.

9. Künstliches kontraktiles Gewebe nach einem der vorhergehenden Ansprüche, wobei der Aktivator ein elektrischer Strom/ eine elektrische Spannung ist.

## Revendications

1. Tissu contractile artificiel, comprenant une structure rigide (b) formant une ligne fermée et plusieurs fibres (a) de longueur variable qui sont fixées à leurs extrémités à deux points séparés de ladite structure (b) et qui sont réparties à travers ladite structure (b) de façon à créer un dôme ; lesdites fibres (a) étant réalisées à partir d'un matériau contractile qui peut être activé par un activateur de façon à fournir un tissu dans une position de repos ou dans une position activée, la position de repos étant définie par des fibres (a) non rectilignes, et la position activée étant définie par des fibres (a) de longueur réduite ; la transition de la position de repos à la position activée ou vice versa étant définie par un mouvement de fibre le long d'une direction latérale qui est perpendiculaire par rapport à la longueur de fibre, de sorte que le dôme se rapproche de ladite structure (b).

2. Tissu contractile artificiel selon la revendication 1, dans lequel ladite ligne fermée est comprise dans un plan.

3. Tissu contractile artificiel selon l'une quelconque des revendications précédentes, dans lequel ladite structure (b) présente une forme annulaire, chaque fibre (a) formant un diamètre de ladite structure annulaire (b).

4. Tissu contractile artificiel selon l'une quelconque des revendications précédentes 1 à 2, dans lequel ladite structure (b) présente une forme annulaire, chaque fibre (a) passant dans une rainure (i) d'un pivot central (j) et formant une boucle à travers ledit pivot (j).

5. Tissu contractile artificiel selon l'une quelconque des revendications précédentes, comprenant en outre une membrane (d) qui couvre les fibres (a) d'un côté du tissu.

6. Tissu contractile artificiel selon la revendication 5, comprenant une autre membrane qui couvre l'autre côté du tissu.

7. Tissu contractile artificiel selon l'une quelconque des revendications précédentes 2 à 6, dans lequel, dans une position de repos, ledit plan forme un angle de 20 à 35° avec les extrémités de fibre.

8. Tissu contractile artificiel selon l'une quelconque des revendications précédentes, dans lequel la surface externe de la structure (b) comprend une surface de couture (e).

9. Tissu contractile artificiel selon l'une quelconque des revendications précédentes, dans lequel ledit activateur est un courant/une tension électrique.
